(19) Europäisches Patentamt European Patent Office Office européen des brevets

(11) **EP 4 678 102 A1**

(12) **EUROPEAN PATENT APPLICATION**

(43) Date of publication:
**14.01.2026 Bulletin 2026/03**

(21) Application number: 24188383.4

(22) Date of filing: **12.07.2024**

(51) International Patent Classification (IPC):
***A61B 5/1455*** (2006.01)     ***A61B 5/00*** (2006.01)

(52) Cooperative Patent Classification (CPC):
**A61B 5/14552; A61B 5/1455; A61B 5/6814; A61B 5/6826**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **PAULUSSEN, Elvira Johanna Maria**
  **Eindhoven (NL)**
• **DAMINK, Paul**
  **Eindhoven (NL)**
• **VAN DEN DUNGEN, Wilhelmus Andreas Marinus**
  **Arnoldus**
  **Maria**
  **5656AG Eindhoven (NL)**

(74) Representative: **Philips Intellectual Property & Standards**
**High Tech Campus 52**
**5656 AG Eindhoven (NL)**

(54) **PHOTOPLETHYSMOGRAPHY SENSOR**

(57) Proposed concepts thus aim to provide schemes, solutions, concepts, designs, methods, and systems pertaining to a PPG sensor system comprising an optical source arrangement, a reflector, and an optical detector arrangement for measuring oxygen saturation of blood within bodily tissue. In particular, embodiments aim to provide a PPG sensor system configured such that optical radiation emitted by the optical source arrangement is received at the optical detector arrangement via both transmissive and reflective ray paths through bodily tissue.

FIG. 1

EP 4 678 102 A1

**Description**

FIELD OF THE INVENTION

This invention relates to the field of Photoplethysmography (PPG) sensors and more particularly to PPG sensor systems for measuring oxygen saturation.

BACKGROUND OF THE INVENTION

PPG measurements may be used to easily obtain vital sign measurements. The principle involves the use of optical measurements to detect blood volume changes that may then be used to determine values such as heart rate, respiration rate, and blood oxygen saturation ($SpO_2$). PPG sensors therefore typically involve the emission of optical radiation to a bodily tissue and the detection of this optical radiation once it has interacted with the bodily tissue.

Pulse oximeter sensors are a type of PPG sensor used to measure the oxygen saturation level in a patient's blood. In pulse oximeter sensors, light travels through the tissue and is then detected by a photodiode. The light that travels through the tissue can be categorized into functional light and non-functional light. Functional light is light that contains pulsatile information from blood volume change in the cardiac cycle. Non-functional light does not contain pulsatile information and can introduce calibration artifacts in pulse oximeter sensors. This includes shunt light that did not pass through the tissue and DC light that does pass through the tissue but doesn't contain any pulsatile information.

PPG sensors used for pulse oximetry typically come in two forms, transmission-based sensors and reflection-based sensors. Transmission-based sensors measure light that has been transmitted through the full thickness of the patient's tissue (for example, by placing a light detector on the opposite side of the tissue to the light detector) whereas reflection-based sensors measure light that is internally scattered/reflected within the patient's tissue and therefore does not traverse the tissue's full thickness (for example, by placing a light detector on the same side of the tissue as a light source). Both forms of sensor face issues when detecting optical radiation in thin tissue. To achieve a high signal-to-noise ratio (SNR) of the measurements it is necessary that the modulation depth associated with the measured optical radiation be high. In typical transmission-based sensors this distance is limited by the thickness of the tissue being measured. In typical reflection-based sensors, the light source and the light detector must be placed sufficiently far apart to achieve a high modulation depth. This places constraints on how small the pulse oximeter can be.

SUMMARY OF THE INVENTION

The invention is defined by the claims.

According to examples in accordance with an aspect of the invention, there is provided a PPG sensor system for measuring oxygen saturation of blood within bodily tissue.

The system comprises: a support structure configured, in use, to receive bodily tissue; an optical source arrangement coupled to the support structure and configured to emit optical radiation; a reflector coupled to the support structure and configured to reflect optical radiation incident on the reflector; and an optical detector arrangement configured to detect optical radiation incident on the optical detector arrangement, wherein the support structure is configured such that, in use the optical source arrangement and the optical detector arrangement are positioned at a first side of the bodily tissue and the reflector is positioned at a second side of the bodily tissue opposite the first side, and wherein the optical source arrangement and the reflector are adapted such that, in use, optical radiation emitted by the optical source arrangement is received at the optical detector arrangement via both transmissive and reflective ray paths through the bodily tissue.

Proposed concepts thus aim to provide schemes, solutions, concepts, designs, methods, and systems pertaining to a PPG sensor system comprising an optical source arrangement, a reflector, and an optical detector arrangement for measuring oxygen saturation of blood within bodily tissue. In particular, embodiments aim to provide a PPG sensor system configured such that optical radiation emitted by the optical source arrangement is received at the optical detector arrangement via both transmissive and reflective ray paths through bodily tissue.

Typically, pulse oximeter sensors are configured to detect either transmissive ray paths (for example by positioning an optical detector on an opposite side of the tissue to the optical radiation source such that optical radiation that is transmitted through the full thickness of the tissue may be measured) or reflective ray paths (for example by positioning an optical detector on the same side of the tissue as the optical radiation source such that it detects optical radiation that is internally reflected within the bodily and therefore emerges from the tissue at the same side at which it entered the bodily tissue). When measuring thin tissues each of these techniques may suffer from significant degradation of the measured signal since the modulation depth of the detected radiation is decreased due to the thinness of the tissue. In the case of reflective pulse oximetry, this can in part be overcome by increasing the separation between the optical detector and the optical source, however this will result in a lower intensity of radiation being detected and result in minimum size constraints on the device containing the sensor system.

In the PPG sensor system of the proposed invention, a reflector is configured to lie on the opposite side of the bodily tissue to the optical source and the optical detector when the sensor system is in use. The reflector may therefore reflect optical radiation that has traversed the full thickness of the bodily tissue (passed through the tissue via transmissive ray paths) from the optical source arrangement back towards the optical detector arrangement. In this way the proposed system is configured to leverage both reflective and transmissive ray paths through the bodily tissue. This may result in an increase in signal level by the increase of light paths and increased signal-to-noise ratio of a generated signal based on the detected optical radiation allowing for more accurate monitoring and assessment of bodily tissues and in particular improved determination of the blood oxygen saturation of bodily tissues.

In some embodiments, the optical detector arrangement may comprise a plurality of optical detectors each of the plurality of optical detectors configured, in use, to be positioned at a different distance from the optical source arrangement. The use of multiple detectors may allow for the detection of optical radiation associated with different penetration depths within the tissue and therefore may allow for an optimal detection position for detecting both transmissive and reflective ray paths to be identified.

In some embodiments, the optical source arrangement may be configured to emit optical radiation within a plurality of different predetermined wavelength ranges, and each of the plurality of optical detectors may be configured to detect optical radiation within a different predetermined wavelength range. Some PPG sensing methods rely on the use of multiwavelength measurements, particularly in the case of pulse oximetry. The ray paths associated with different wavelengths of radiation may differ due to the different optical properties of the bodily tissue at different wavelengths. The use of multiple detectors at different distances where each detector detects a different wavelength of optical radiation therefore allows for the detection of transmissive and reflective ray paths for each wavelength.

In some embodiments, the optical source arrangement comprises a plurality of optical sources and wherein each of the plurality of optical sources emits optical radiation within a different wavelength range. This may improve control in multiwavelength measurement schemes.

In some embodiments, at least one of the optical source arrangement and the reflector are configured to have an adjustable parameter such that the reflective and/or transmissive ray paths of optical radiation received at the optical detector arrangement can be adjusted. This configuration allows for a greater degree of control over the ray paths taken by detected optical radiation to allow for optimization of the SNR.

In some embodiments, an adjustable parameter of the optical source arrangement may comprise at least one of: an orientation of the optical source arrangement; an emission angle of the optical source arrangement; the wavelength of optical radiation emitted by the optical source arrangement; the position of the optical source arrangement, in use, relative to the position of the reflector; and the position of the optical source arrangement, in use, relative to the position of the optical detector arrangement. The adjustment of any of these parameters of the optical source arrangement may permit the adjustment of the ray paths of optical radiation detected at the optical detector arrangement such that both transmissive and reflective ray paths are detected.

In some embodiments, an adjustable parameter of the reflector may comprise at least one of: an orientation of the reflector; the wavelength of optical radiation reflected by the reflector; the size of the reflector; the shape of the reflector; the position of the reflector, in use, relative to the position of the optical source arrangement; and the position of the reflector, in use, relative to the position of the optical detector arrangement. Each of these parameters will affect the transmissive ray paths taken by optical radiation from the optical source arrangement to the optical detector arrangement and therefore their adjustment permits the adaption of the PPG sensor system such that both reflective and transmissive ray paths are detected at the optical detector arrangement.

In some embodiments, the PPG sensor system may be configured such that, in use, a distance between the optical source arrangement and the optical detector arrangement is greater than or substantially equal to twice the thickness of the bodily tissue. This geometry provides one way of ensuring that both reflective and transmissive ray paths are detected at the optical detector arrangement, resulting in an improved signal-to-noise ratio.

In some embodiments, the optical detector arrangement and the optical source arrangement may comprise an LED light source that acts as both the optical detector arrangement and the optical source arrangement. This may be advantageous in reducing the size of the PPG sensor system and simplifying its manufacture.

According to another aspect of the proposed invention, there is provided a pulse oximeter device comprising the PPG sensor system of any of the above proposed embodiments.

According to yet another aspect of the proposed invention, there is provided a system for measuring oxygen saturation of blood within bodily tissue, the system comprising: the pulse oximeter described above; and a processing arrangement configured to: receive from the pulse oximeter an optical radiation detection signal describing optical radiation detected by the optical detector arrangement of the pulse oximeter; and process the optical radiation detection signal to determine an oxygen saturation value.

There is further provided a method for operating a PPG sensor system, the PPG sensor system comprising: a support structure configured, in use, to receive bodily tissue; an optical source arrangement coupled to the support structure and configured to emit optical radiation; a reflector coupled to the support structure and configured to reflect optical radiation incident on the reflector; and an optical detector arrangement coupled to the support structure and configured to detect optical radiation incident on the optical detector arrangement. The method comprises: configuring the support structure of the PPG sensor system such that, in use, the optical source arrangement and the optical detector arrangement are positioned at a first side of the bodily tissue and the reflector is positioned at a second side of the bodily tissue opposite the first side; and adapting the optical source arrangement and reflector such that, in use, optical radiation emitted by the optical source arrangement is received at the optical detector arrangement via both transmissive and reflective ray paths through the tissue of the subject.

Embodiments may be employed in combination with conventional/existing PPG sensing methods and systems. In this way, embodiments may integrate into legacy systems to improve and/or extend their functionality and capabilities. An improved PPG sensor system that allows for the determination of oxygen saturation in blood based on detected optical radiation may therefore be provided by the proposed embodiments.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a simplified illustration of a PPG sensor system according to a proposed embodiment;
Fig. 2 shows a simplified illustration of a PPG sensor system according to another proposed embodiment;
Fig. 3 shows a simplified illustration of a PPG sensor system according to a third proposed embodiment;
Fig. 4 depicts a simplified block diagram of a system for measuring oxygen saturation of blood within bodily tissue according to an aspect of the proposed invention; and
Fig. 5 depicts a simplified flow diagram of a method for operating a PPG sensor system according to a proposed embodiment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The proposed invention provides a system and method for PPG sensing that may be particularly advantageous for thin tissue thicknesses such as the ear lobe, nostril or small fingers. The system and method are designed to enhance the signal-to-noise ratio (SNR) and achieve more accurate readings of PPG signals by combining reflection- and transmission-based measurements.

Different sensor configurations are possible for PPG sensors used to measure oxygen saturation. Transmission-based configurations rely on detecting optical radiation that has traversed the full thickness of a bodily tissue. This may be achieved, as just one example, by positioning an optical detector on the opposite side of a bodily tissue to an optical source and measuring the radiation that passes through the tissue. In this instance, the thickness of the bodily tissue determines the intensity and the modulation depth of the detected optical radiation. The modulation depth relates to measured variations in the intensity level of the optical radiation at different points in the cardiac cycle. It is typically defined as

$$M = \frac{P_{dia} - P_{sys}}{Average(P_{dia}, P_{sys})},$$

$$(1)$$

where $P_{dia}$ is the measured optical power at diastole of the cardiac system and $P_{sys}$ is the measured optical power at systole of the cardiac system. To achieve accurate interpretation of the PPG signals it is important that both the intensity of the measured optical radiation and the modulation depth are sufficiently high.

Reflection-based PPG sensors rely on detecting optical radiation that has been reflected by the bodily tissue such that it emerges from the bodily tissue on the same side of the bodily tissue that it was incident on. This may be achieved by positioning an optical detector on the same side of the bodily tissue as the optical source. In this instance, the larger the distance between the optical source and the optical detector the larger the penetration depth of the photons into the bodily tissue and the weaker the intensity of the detected radiation. Large penetration depth is beneficial as it results in a larger modulation depth, however this comes with the drawback of a lower radiation intensity incident on the optical detector arrangement and therefore a lower signal-to-noise ratio.

The proposed invention is beneficial since it allows for the detection of both reflective and transmissive ray paths in order to improve the signal-to-noise ratio. A reflector is used to reflect transmitted light that has passed through a bodily tissue towards the optical detector such that transmissive ray paths can also be detected at an optical detector placed on the same side of the bodily tissue as the optical source. This may be particularly advantageous for the case of thin tissue measurements for which it may be difficult to achieve a high detection intensity and a high modulation depth for achieving a high SNR.

When measuring both reflective and transmissive ray paths the total measured optical power at the optical detector arrangement is given by the sum of the power of each individual ray path:

$$P_{tot} = \sum_{i=1}^{N} P_i .$$

$$(2)$$

The modulation depth then becomes:

$$(3)$$

$$M_{tot} = \frac{\sum_{i=1}^{N} P_{dia,i} - \sum_{i=1}^{N} P_{sys,i}}{Average(\sum_{i=1}^{N} P_{dia,i}, \sum_{i=1}^{N} P_{sys,i})},$$

such that the overall modulation depth when detecting both reflective and transmissive ray paths is greater than when only measuring one of reflective or transmissive ray paths..

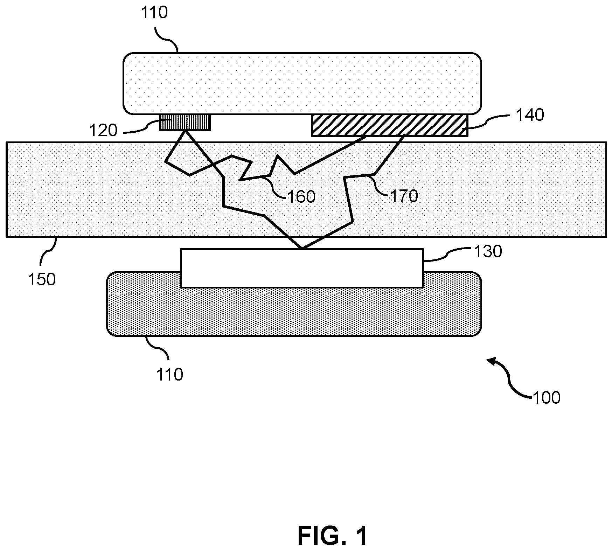

Referring now to Fig. 1, there is depicted a simplified illustration of a PPG sensor system 100 according to a proposed embodiment.

The PPG sensor system 100 comprises a support structure 110 that is configured to receive bodily tissue 150. In particular, the support structure 110 is configured to accommodate a bodily tissue of thin thickness such as an ear lobe, a nostril, or a small finger (such as that of an infant).

The PPG sensor system 100 further comprises an optical source arrangement 120 coupled to the support structure 110. In use, the support structure is configured such that the optical source arrangement is positioned at a first side of the bodily tissue. The optical source arrangement emits optical radiation to the bodily tissue.

A reflector 130 and an optical detector arrangement 140 are also couped to the support structure 110. The reflector 130 is configured to reflect optical radiation incident on the reflector and the optical detector arrangement 140 is configured to detect optical radiation incident on the optical detector arrangement.

The support structure 110 is configured such that, in use, the optical source arrangement 120 and the optical detector arrangement 130 is positioned on a first side of the bodily tissue 150, and the reflector is positioned at a second side of the bodily tissue opposite the first side. In this way, the reflector may enhance the intensity detected by the optical detector arrangement by reflecting optical radiation emitted by the optical source arrangement that is transmitted through the full thickness of the bodily tissue towards the optical detector arrangement as shown by the transmissive ray path 170 in Fig. 1.

The optical source arrangement is further configured such that the optical detector arrangement also detects optical radiation that has passed through the bodily tissue from the optical source arrangement 120 via reflective ray paths such as the ray path 160 shown in Fig. 1. Thus, the proposed PPG sensor system is advantageous in that it allows for the detection of both reflective and transmissive ray paths of optical radiation through a bodily tissue. This may result in an enhanced detected radiation and an increased modulation depth for the same thickness of tissue. As such a signal generated by the PPG sensor system 100 may have an improved signal-to-noise ratio compared to a PPG sensor configured to detect only reflective or only transmissive ray paths through the tissue. The reflective ray path 160 and the transmissive ray path 170 shown in Fig. 1 are just two examples of possible ray paths through the tissue 150. Many other transmissive and reflective ray paths from the optical source arrangement to the optical detector arrangement exist.

Fig. 1 shows just one possible configuration of the support structure 110. In other embodiments, the support structure maybe configured differently with any suitable combination of flexible and rigid elements to ensure that, in use, the optical source arrangement and the optical detector arrangement are positioned at a first side of the bodily tissue and the reflector is positioned at a second side of the bodily tissue opposite the first side.

Although in Fig. 1, the optical source arrangement and the optical detector arrangement are shown as separate components, this need not be the case in other embodiments. In some embodiments, the optical detector arrangement and the optical source arrangement comprise an LED optical radiation source that acts as both the optical detector arrangement and the optical source arrangement. This may be advantageous in reducing the size of the PPG sensor system.

In some embodiments, the optical source arrangement 120 is configured to emit optical radiation within a predetermined wavelength range that is selected based on the bodily tissue being analyzed and the intended use of the detected optical radiation measurements. The reflector 130 and optical detector arrangement 140 may be similarly configured to only reflect and detect optical radiation within the predetermined wavelength range.

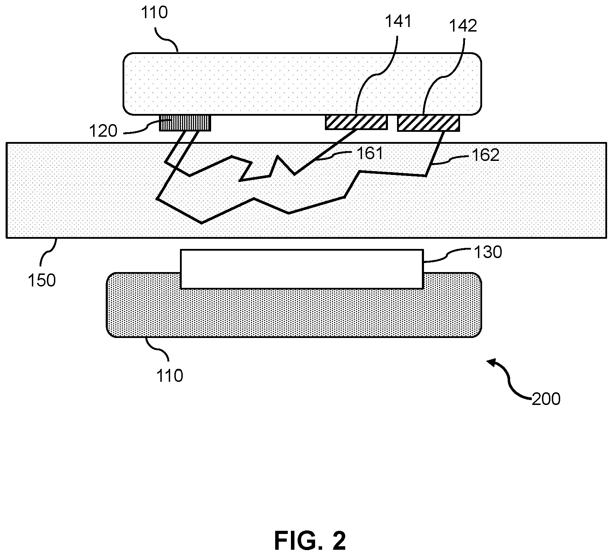

Referring now to Fig. 2, there is depicted a PPG sensor system 200 according to another proposed embodiment.

Similar to the PPG sensor system 100, the PPG sensor system 200 comprises a support structure 110, an optical source arrangement 120 and a reflector 130. These components are similar to the components discussed in reference to Fig. 1 and therefore a detailed description of these components will not be repeated.

The PPG sensor system 200 differs from the PPG sensor system 100 in that the optical detector arrangement 140 in this embodiment comprises a plurality of optical detectors, specifically a first optical detector 141 and a second optical detector 142. Each of these optical detectors 141, 142 is configured to be positioned at a different distance from the optical source arrangement 120.

In this exemplary embodiment, the optical source arrangement 120 is configured to emit optical radiation within a plurality of different predetermined wavelength ranges. In this instance, each of the optical detectors 141 and 142 is configured to detect optical radiation within a different predetermined wavelength range. This may be achieved via proper filtering components at each of the optical detectors but other techniques in the art may be used. Ray paths through the bodily tissue 150 will depend on the wavelength of the optical radiation as the optical properties of the tissue will vary with wavelength. For example, Fig. 2 shows a first reflective ray path 161 through the bodily tissue corresponding to optical radiation of a first wavelength and a second reflective ray path 162 through the bodily tissue corresponding to optical radiation of a second wavelength. By configuring optical detectors at different distances from the optical source arrangement to detect radiation of a different wavelength, the system may be configured to ensure that both reflective and transmissive ray paths are measured for all wavelengths and that therefore the SNR of the detected radiation is low for all detected wavelengths. The use of multi-wavelength measurements may be useful for particular applications of PPG sensor systems and in particular for pulse oximetry.

Typical measurements of oxygen saturation ($SpO_2$) using PPG sensor systems involve the emission of optical radiation of at least two different wavelengths that are selected such that the molar absorption coefficients of oxygenated haemoglobin and deoxygenated haemoglobin at these both of these wavelengths are well separated. Typically, red light and infrared radiation are used. By comparing the intensity of detected radiation associated with each wavelength the oxygen saturation of blood (the SpOz value) may be derived.

Although in Fig. 2 the optical source arrangement 120 is shown as a single component, in some embodiments, the optical source arrangement 120 may comprise a plurality of optical sources, wherein each of the plurality of optical sources emits optical radiation within a different wavelength range. Switching mechanisms may further be provided such that it is possible to control at any given time which of the plurality of optical sources is emitting optical radiation. Although only two optical detectors are shown in Fig. 2, in other embodiments the optical detector arrangement may comprise more than two optical detectors. In particular, in some embodiments the optical source arrangement may comprise a two-dimensional array of optical detectors.

Fig. 2 depicts a PPG sensor system 220 for obtaining multi-wavelength measurements, however the principal of multiple optical sources and/or multiple optical detectors may equally well be applied to configurations in which only single wavelength ranges are used. This may be beneficial in permitting the detection of a greater range of ray paths through the bodily tissue and in particular ray paths associated with different penetration depths within the bodily tissue 150.

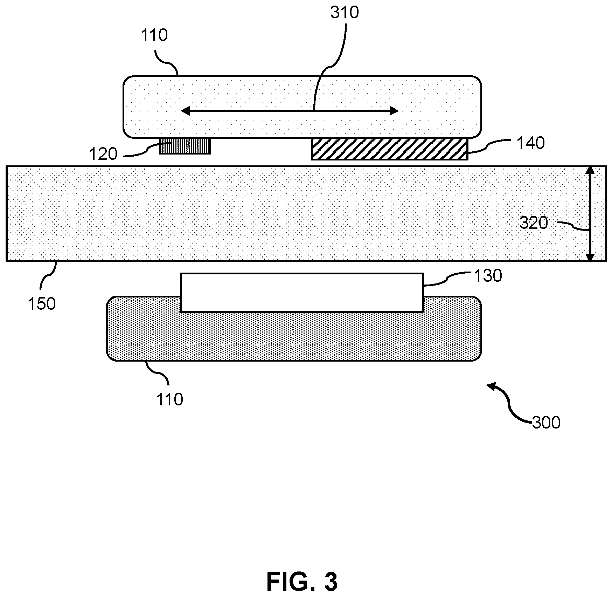

Referring now to Fig. 3, there is depicted a PPG sensor 300 arrangement according to another proposed embodiment.

In this embodiment, the support structure is configured such that the distance between the optical source arrangement 120 and the optical detector arrangement 140 (indicated by the arrow 310) is greater than or substantially equal to twice the thickness of the bodily tissue 150 (indicated by the arrow 320). This configuration can allow for the combination of reflective and transmissive measurements, which can enhance the signal-to-noise ratio and improve the accuracy of oxygen saturation readings.

Further, in this exemplary embodiment, the support structure 110 is configured such that positioning of the optical source arrangement 120, the optical detector arrangement 140, and the reflector 130 in relation to the bodily tissue 150 can be adjusted to optimize the performance of the PPG sensor system 10, whilst maintaining the design rule outlines above. For example, the distance between the optical source arrangement 120 and the optical detector arrangement 140 can be adjusted to optimize the path of the optical radiation through the bodily tissue 150, and the position of the reflector 130 can be adjusted to optimize the reflection of the optical radiation back towards the optical detector arrangement 140. In particular, the distance between the optical source arrangement 120 and the optical source arrangement 130 may be adjusted depending on the thickness of the bodily tissue 150 such that this distance is always greater than or equal to twice the thickness of the bodily tissue.

In other embodiments, other parameters of the PPG sensor system may be adjustable. For example, at least one of the optical source arrangement and the reflector may be configured to have an adjustable parameter such that the reflective and/or transmissive ray paths of optical radiation received at the optical detector arrangement can be modified. This may allow for the configuration and fine tuning of the PPG sensor system such that it reliably detects both transmissive and reflective ray paths through the bodily tissue.

An adjustable parameter of the optical source arrangement may comprise at least one of: an orientation of the optical source arrangement; an emission angle of the optical source arrangement; the wavelength of the optical radiation emitted by the optical source arrangement; the position of the optical source arrangement, in use, relative to the position of the reflector; and the position of the optical source arrangement, in use, relative to the position of the optical detector arrangement. An adjustable parameter of the reflector may comprise at least one of: an orientation of the reflector; the wavelength of optical radiation reflected by the reflector; the size of the reflector; the shape of the reflector; the position of the reflector, in use, relative to the position of the optical source arrangement; and the position of the reflector, in use, relative to the position of the optical detector arrangement.

Key examples embodiments of the proposed invention may therefore involve adjusting the path optical radiation takes through the bodily tissue by adjusting parameters of the components of the PPG sensor system. In some embodiments, a large range of ray paths may be scanned when the PPG sensor system is in use by adjusting the direction in which the optical source arrangement emits optical radiation or the orientation of the reflector. The optimal positioning and configuration of the optical source arrangement and the reflector may therefore be selected by analyzing the measured radiation at the optical source detector as the different ray paths are scanned. In other embodiments, the size of the reflector 130 may be adjustable and may be adjusted depending on the wavelength of the optical radiation and/or optical source that the optical detector arrangement is configured to detect.

Some embodiments may involve adjusting multiple parameters of the optical source arrangement and/or the reflector sequentially to optimize the detection of reflective and transmissive ray paths at each detector of the optical detector arrangement for each source of the optical source arrangement. In embodiments in which the optical source arrangement comprises a plurality of different optical source arrangements and is configured to emit optical radiation within a plurality of different wavelength ranges, the adjustment of parameters of the optical source arrangement and reflector arrangements can be synchronized with a wavelength or source modulation at the optical source arrangement in order to independently optimize the ray paths for each source and/or wavelength.

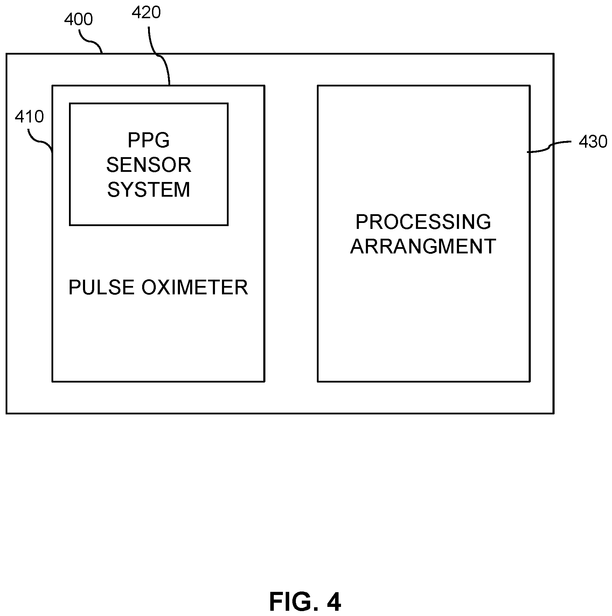

Referring now to Fig. 4, there is depicted a simplified block diagram of a system 400 for measuring oxygen saturation of blood in bodily tissue. The system 400 comprises a pulse oximeter device 410 which houses the PPG sensor system 100. The pulse oximeter device is designed to detect and measure the oxygen saturation levels in the blood. The PPG sensor system 100, as previously described, comprises an optical source arrangement 120, a reflector 130, and an optical detector arrangement 140, all configured to interact with bodily tissue in a manner that allows for the combination of reflective and transmissive measurements.

The system 400 further comprises a processing arrangement 430. The processing arrangement is configured to process data received from the PPG sensor system to determine an oxygen saturation value. Specifically, the processing arrangement 430 is configured to receive an optical radiation detection signal from the pulse oximeter device 410. This signal describes the optical radiation detected by the optical detector arrangement 140 which has passed through bodily tissue via both reflective and transmissive ray paths. The processing arrangement is configured to process this signal and determine an oxygen saturation value.

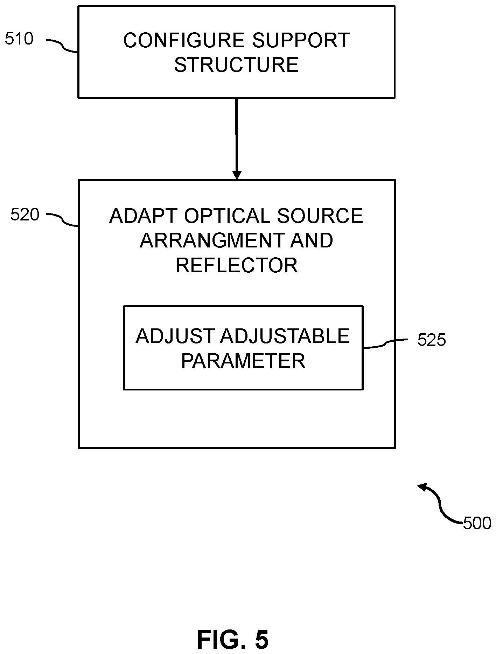

Referring now to Fig. 5, there is depicted a simplified flow diagram of a method 500 for operating a PPG sensor system. The PPG sensor system in question comprises a support structure, an optical source arrangement coupled to the support structure, a reflector coupled to the support structure, and an optical detector arrangement coupled to the support structure.

The method commences with a step 510 comprising configuring the support structure of the PPG sensor system such that, in use, the optical source arrangement and the optical detector arrangement are positioned at a first side of the bodily tissue and the reflector is positioned at a second side of the bodily tissue opposite the first side.

The method then proceeds to a step 520 comprising adapting the optical source arrangement and the reflector such that, in use, optical radiation emitted by the optical source arrangement is received at the optical detector arrangement via both transmissive and reflective ray paths through the tissue of the subject.

In this embodiment, the step 520 comprises the sub-step 525 of adjusting an adjustable parameter of at least one of the optical source arrangement and the reflector to modify the reflective and/or transmissive ray paths of optical radiation received at the optical detector arrangement.

Thus, the proposed invention may further provide a novel method for operating PPG sensor systems that allows for accurate determination of blood oxygen saturation levels.

Variations to the disclosed embodiments can be understood and effected by those skilled in the art in practicing the claimed invention, from a study of the drawings the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If the term "adapted to" is used in the claims or description it is noted the term "adapted to" is intended to be equivalent to the term "configured to". Any reference signs in the claims should not be construed as limiting the scope.

**Claims**

1. A PPG sensor system (100) for measuring oxygen saturation of blood within bodily tissue, the system comprising:

   a support structure (110) configured, in use, to receive bodily tissue;
   an optical source arrangement (120) coupled to the support structure and configured to emit optical radiation;
   a reflector (130) coupled to the support structure and configured to reflect optical radiation incident on the reflector; and
   an optical detector (140) arrangement coupled to the support structure and configured to detect optical radiation incident on the optical detector arrangement,
   wherein the support structure is configured such that, in use, the optical source arrangement and the optical detector arrangement are positioned at a first side of the bodily tissue and the reflector is positioned at a second side of the bodily tissue opposite the first side, and
   wherein the optical source arrangement and the reflector are adapted such that, in use, optical radiation emitted by the optical source arrangement is received at the optical detector arrangement via both transmissive (170) and reflective (160) ray paths through the bodily tissue.

2. The PPG sensor system of claim 1, wherein the optical detector arrangement comprises a plurality of optical detectors (141, 142), each of the plurality of optical detectors configured, in use, to be positioned at a different distance from the optical source arrangement.

3. The PPG sensor system of claim 2, wherein the optical source arrangement is configured to emit optical radiation within a plurality of different predetermined wavelength ranges, and
   wherein each of the plurality of optical detectors (141, 142) is configured to detect optical radiation within a different predetermined wavelength range.

4. The PPG sensor system of any preceding claim, wherein the optical source arrangement comprises a plurality of optical sources, and wherein each of the plurality of optical sources emits optical radiation within a different wavelength range.

5. The PPG sensor system of any preceding claim, wherein at least one of the optical source arrangement and the reflector are configured to have an adjustable parameter such that the reflective and/or transmissive ray paths of optical radiation received at the optical detector arrangement can be modified.

6. The PPG sensor system of claim 5, wherein an adjustable parameter of the optical source arrangement comprises at least one of:

   an orientation of the optical source arrangement; a direction in which the optical source arrangement is configured to emit optical radiation;
   an emission angle of the optical source arrangement;
   the wavelength of optical radiation emitted by the optical source arrangement;
   the position of the optical source arrangement, in use, relative to the position of the reflector; and
   the position of the optical source arrangement, in use, relative to the position of the optical detector arrangement.

7. The PPG sensor system of any of claims 2 and 3, wherein an adjustable parameter of the reflector comprises at least one of:

   an orientation of the reflector; a direction in which the reflector is configured to reflect optical radiation;

the wavelength of optical radiation reflected by the reflector;
the size of the reflector;
the shape of the reflector;
the position of the reflector, in use, relative to the position of the optical source arrangement; and
the position of the reflector, in use, relative to the position of the optical detector arrangement.

8. The PPG sensor system of any preceding claim wherein the system is configured such that, in use, a distance between the optical source arrangement and the optical detector arrangement (310) is greater than or substantially equal to twice the thickness (320) of the bodily tissue.

9. The PPG sensor system of any preceding claim, wherein the optical detector arrangement and the optical source arrangement comprise an LED optical radiation source that acts as both the optical detector arrangement and the optical source arrangement.

10. A pulse oximeter device (410) comprising the PPG sensor system of any of claims 1-9.

11. A system (400) for measuring oxygen saturation of blood within bodily tissue, the system comprising:

the pulse oximeter device (410) of claim 10; and
a processing arrangement (430) configured to:

receive from the pulse oximeter an optical radiation detection signal describing optical radiation detected by the optical detector arrangement of the pulse oximeter; and
process the optical radiation detection signal to determine an oxygen saturation value.

12. A method (500) for operating a PPG sensor system, the PPG sensor system comprising: a support structure configured, in use, to receive bodily tissue;

an optical source arrangement coupled to the support structure and configured to emit optical radiation;
a reflector coupled to the support structure and configured to reflect optical radiation incident on the reflector; and
an optical detector arrangement coupled to the support structure and configured to detect optical radiation incident on the optical radiation detector arrangement,
the method comprising:

configuring (510) the support structure of the PPG sensor system such that, in use, the optical source arrangement and the optical detector arrangement are positioned at a first side of the bodily tissue and the reflector is positioned at a second side of the bodily tissue opposite the first side, and
adapting (520) the optical source arrangement and the reflector such that, in use, optical radiation emitted by the optical source arrangement is received at the optical detector arrangement via both transmissive and reflective ray paths through the tissue of the subject.

13. The method of claim 12, wherein adapting the optical source arrangement and the reflector comprises adjusting (525) an adjustable parameter of at least one of the optical source arrangement and the reflector to modify the reflective and/or transmissive ray paths of optical radiation received at the optical detector arrangement.

14. The method of claim 13, wherein an adjustable parameter of the optical source arrangement comprises at least one of:

an orientation of the optical source arrangement;
an emission angle of the optical source arrangement;
the wavelength of optical radiation emitted by the optical source arrangement;
the position of the optical source arrangement, in use, relative to the position of the reflector; and
the position of the optical source arrangement, in use, relative to the position of the optical detector arrangement.

15. The method of any of claims 13 and 14, wherein an adjustable parameter of the reflector comprises at least one of:

an orientation of the reflector;
the wavelength of optical radiation reflected by the reflector;
the size of the reflector;

the shape of the reflector;
the position of the reflector, in use, relative to the position of the optical source arrangement; and
the position of the reflector, in use relative to the position of the optical detector arrangement.

**FIG. 1**

**FIG. 2**

**FIG. 3**

**FIG. 4**

510 — CONFIGURE SUPPORT STRUCTURE

520 — ADAPT OPTICAL SOURCE ARRANGMENT AND REFLECTOR

ADJUST ADJUSTABLE PARAMETER — 525

500

FIG. 5

Europäisches
Patentamt
European
Patent Office
Office européen
des brevets

# EUROPEAN SEARCH REPORT

**Application Number**

EP 24 18 8383

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/100498 A1 (ZARBEL DOR [IL] ET AL) 8 April 2021 (2021-04-08) * the whole document * | 1-15 | INV. A61B5/1455 A61B5/00 |
| A | US 2017/303830 A1 (KLEIN RONNIE [IL] ET AL) 26 October 2017 (2017-10-26) * paragraph [0007] - paragraph [0028] * * paragraph [0173] * * figures 1-12 * | 1-15 | |
| A | US 2020/367767 A1 (SULLIVAN THOMAS J [US] ET AL) 26 November 2020 (2020-11-26) * paragraph [0006] - paragraph [0032] * * paragraph [0090] * * figure 7 * | 1-15 | |
| A | WO 2016/178986 A1 (LIFEQ GLOBAL LTD [IE]; OLIVIER LAURENCE RICHARD [US] ET AL.) 10 November 2016 (2016-11-10) * the whole document * | 1-15 | |
| A | US 2016/353997 A1 (YODH ARJUN G [US] ET AL) 8 December 2016 (2016-12-08) * paragraph [0007] - paragraph [0033] * * paragraph [0258] * * figure 1 * | 1-15 | TECHNICAL FIELDS SEARCHED (IPC) A61B |

The present search report has been drawn up for all claims

| Place of search | Date of completion of the search | Examiner |
|---|---|---|
| Berlin | 7 November 2024 | Abraham, Volkhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 8383

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2024

| Patent document cited in search report | Publication date | Patent family member(s) | | Publication date |
|---|---|---|---|---|
| US 2021100498 A1 | 08-04-2021 | NONE | | |
| US 2017303830 A1 | 26-10-2017 | NONE | | |
| US 2020367767 A1 | 26-11-2020 | CN | 107072538 A | 18-08-2017 |
| | | US | 2017340219 A1 | 30-11-2017 |
| | | US | 2020367767 A1 | 26-11-2020 |
| | | WO | 2016040264 A1 | 17-03-2016 |
| WO 2016178986 A1 | 10-11-2016 | NONE | | |
| US 2016353997 A1 | 08-12-2016 | US | 2016353997 A1 | 08-12-2016 |
| | | US | 2016361017 A1 | 15-12-2016 |
| | | WO | 2015127434 A1 | 27-08-2015 |
| | | WO | 2015127436 A2 | 27-08-2015 |

For more details about this annex : see Official Journal of the European Patent Office, No. 12/82